# EUROPEAN PATENT APPLICATION

(11) **EP 2 612 658 A1**
(43) Date of publication of application: **10.07.2013**
(21) Application number: 12150321.3
(22) Date of filing: 05.01.2012
(51) Int. Cl.: A61K 9/20, A61K 31/4184, A61K 31/54, A61K 45/06

(54) **Pharmaceutical compositions of 4'-[(1,4'dimethyl-2'-propyl[2,6'-bi-1h-benzimidazol]-1'-yl)methyl]-[1,1'-biphenyl]-2-carboxylic acid and is 6-chloro-3,4-dihydro-2h-1,2,4-benzothiadiazine-7-sulfonamide-1,1-dioxide**

(71) Applicant: Laboratorios Lesvi, S.L., 08970 Sant Joan Despí - Barcelona (ES)
(72) Inventor: Diez Martín, Ignacio, 08970 San Joan Despi (ES); González Pérez, Marino, 08970 San Joan Despi (ES); Úbeda Pérez, Carmen, 08970 San Joan Despi (ES)
(74) Representative: Pons Ariño, Maria Purificación

(57) **Abstract**

This invention relates to stable solid immediate release pharmaceutical compositions of 4'-[(1,4'dimethyl-2'-propyl[2,6'-bi-1H-benzimidazol]-1'-yl)methyl]-[1,1'-biphenyl]-2-carboxylic acid, also known as telmisartan and hydrochlorothiazide (HCTZ or HCT), a thiazide diuretic, which also affords dosage uniformity; process thereof and their use in the treatment of hypertension and essential hypertension.

## Description

### FIELD OF THE INVENTION

This invention relates to stable solid, also known as telmisartan and hydrochlorothiazide (HCTZ or HCT), a thiazide diuretic.

### BACKGROUND OF THE INVENTION

Telmisartan is an angiotensin II receptor antagonist developed for the treatment of hypertension and other medical indications as first disclosed in EP 502314 B1. Its chemical name is 4'-[(1,4'dimethyl-2'-propyl[2,6'-bi-1H-benzimidazol]-1'-yl)methyl]-[1,1'-biphenyl]-2-carboxylic acid, having the following structure:

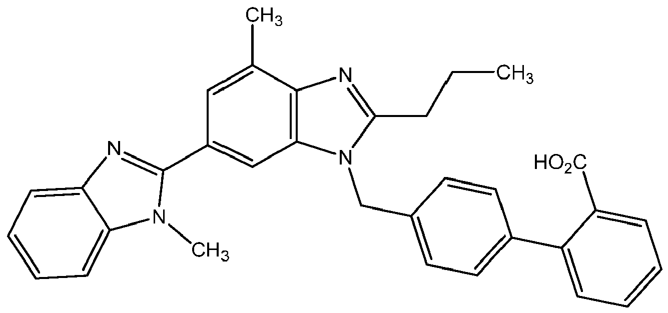

Telmisartan is generally supplied in the free acid form. It is characterised by its very poor solubility in aqueous systems at the physiological pH range of the gastro-intestinal tract of between pH 1 to 7. Two polymorphic forms of telmisartan are known, named crystalline form A and crystalline form B. Crystalline form A is the crystalline form obtained when preparing telmisartan as taught in product patent EP 502314 B1. Crystalline form B is described in EP 1144386 B1.

Hydrochlorothiazide (HCTZ or HCT) is a thiazide diuretic which is orally administered in the treatment of edema and hypertension. Its chemical name is 6-chloro-3,4-dihydro-2H-1,2,4-benzothiadiazine-7-sulfonamide-1,1-dioxide, having the following structure:

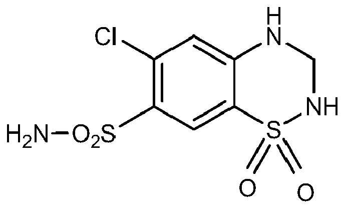

Combinations of telmisartan and hydrochlorothiazide for oral administration are commercialised under the trade name MicardisPlus in three strengths, 40mg/12.5 mg, 80mg/12.5mg and 80mg/25mg of telmisartan/HCTZ.

It is known for the skilled in the art that HCTZ is incompatible with alkaline compounds such as meglumine and sodium hydroxide, which are components of conventional telmisartan/HCTZ formulations. A significant number of patents, for instance WO 2003/059327, WO 2004/028505 and WO 2009/115301, have addressed the incompatibility of HCTZ in basic media and the generation of degradation products. The main degradation product of HCTZ being 4-amino-6-chlorobenzene-1,3-disulphonamide (DSA or impurity B). Formulations containing Telmisartan/HCTZ, prepared as bi-layer tablets by direct compression of each constituent layer, are proposed as a potential solution to overcome the aforementioned incompatibility issues. Although, the interactions between the HCTZ and the basic media were reduced, these bilayer tablets have the disadvantage that its two constituent layers have a propensity to separate because of the lack of a proper bonding of the two layers. In addition, preparing these bilayer tablets requires specific machinery that increases the cost of the final formulation.

WO 2007/144175 discloses a pharmaceutical composition comprising a first unit, containing a first API, among which istelmisartan and a second unit comprising a second API which is HCTZ were prepared. These units could be granules, pellets or tablet cores. Formulations comprising granules containing telmisartan and separated granules containing HCTZ were prepared into a mixture following by compressing them into tablets. Formulations comprising a separating coating applied onto the telmisartan containing unit and/or by incorporating an acidifying substance into the HCTZ containing part of the composition were also disclosed. An important aspect of these two embodiments lies on the incorporation of the binder, polyvinylpyrrolidone, into the HCTZ containing part of the composition and /or in the separating coating, due to its stabilizing properties. Although the impurity profiles were improved versus the prior-art, a high concentration of the main degradation product (DSA), over 0.80%, based on pharmacopeia requirements, were reported for pharmaceutical compositions, comprising telmisartan in the first unit and hydrochlorothiazide in a coating. Pharmaceutical compositions comprising hydroxypropyl methylcellulose and hydroxypropyl cellulose in the separating coating also showed a tenfold increase in the concentration of DSA, in comparison with pharmaceutical compositions comprising polyvinylpyrrolidone in the separating coating, when exposed to stress stability testing at 60 °C for 14 days.

WO2009/058950 described bilayers pharmaceutical formulation prepared by direct compression of the two already prepared layers. Although the impurities profile was improved, these bi-layers formulations prepared by direct compression of the two layers still pose the disadvantages previously explained. Multiparticular tablets, wherein telmisartan and HCTZ were incorporated into the particles, which were bland and compressed together. Moreover, telmisartan containing cores directly coated with HCTZ containing alcoholic coating also comprising hydroxypropil methyl cellulose and polyethylene glycol were prepared. However, no experimental results with regards the stability or performance results of the multiparticular or core coated tablets were reported

As explained above, Telmisartan/HCTZ formulations have been reported to be unstable because of the stability problems of HCTZ in basic mediums, leading over the year to the development of various formulations systems to overcome this issue. Thus, a stable pharmaceutical composition containing telmisartan/HCTZ, which provides dosages uniformity by means of standard and industrially viable excipients and processes is still in need to be developed.

### BRIEF DESCRIPTION OF THE DRAWINGS

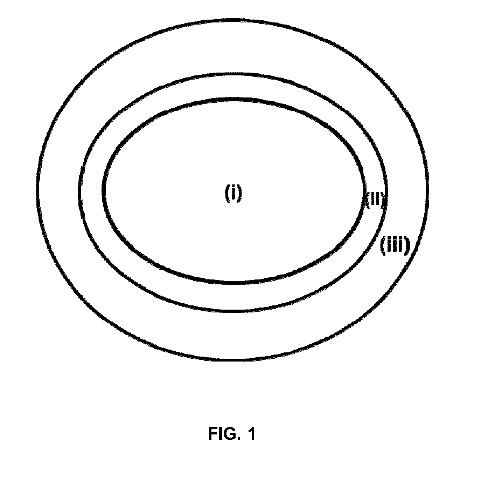

Figure 1 shows the non-proportional section of a tablet, which is a preferred embodiment of the pharmaceutical composition according to the present invention.

### DEFINITIONS

The term "core" as used herein is any compressed solid material that comprises a mixture of telmisartan or salts thereof and acceptable excipients. The term core may include single or multiple tablets, tablet cores, minitablets, capsules, granules, particles, pellets, beads, or beadlets, or any combination thereof. Preferably the core is a tablet or a tablet core. The core may be coated completely or partially with a coating. The shape and size of the cores that can be used in the invention are essentially unlimited.

The term "coating" as used herein refers to adherence, adsorption, loading and/or incorporation, to some extent, preferable uniformly, of at least one solution, dispersion or suspension coating material onto and/or into a substrate. Preferably the coating material is an aqueous dispersion or suspension. The coating material on the substrate may be of any thickness. Preferably the coating material is a thin and uniform film applied onto the substrate.

The term "film coating" as used herein refers to a thin and uniform film applied onto a substrate. Film coating may contain active pharmaceutical ingredients and/or excipients.
The procedures that can be used for film coating are spray or spray-drying by means of coating pan, spray or fluidized coating equipment. Preferably the film coating is performed by spray-drying using a coating pan.

The term "isolating film coating" as used herein refers to a film coating that isolates the core from other components added to the dosage form, preventing contact between pharmaceutical active ingredients and/or excipients.

The term "external film coating" as used herein refers to a film coating that prevents loss of any ingredient of the already functionally complete dosage form and improves the appearance of a pharmaceutical composition.

As used herein, the term "immediate release" refers to the rapid release of the majority of the therapeutic compounds contained in a pharmaceutical composition, allowing said therapeutic compounds to dissolve in the gastrointestinal contents, with no intention of delaying or prolonging the dissolution or absorption of the therapeutic compounds. Preferred conditions for immediate release, as states in European Pharmacopeia (5.17.1, monograph), are the release of at least 80% of the therapeutic compounds within a specified time, typically 45 min or less after oral ingestion. An equivalent term to immediate release is also the term conventional release.

As used herein, the term "sugar" refers to any sugar, including monosaccharides, disacharides and oligosaccharides. Sugars suitable for use in the formulations disclosed herein include monosaccharides such as glucose, dextrose, fructose, mannose, galactose, arabinose, xylose and ribose, etc., and also oligosaccharides such as disaccharides (maltose, lactose, sucrose, trehalose, etc.) and trisaccharides (e.g. raffinose, maltotriose, etc.). The preferred sugar is maltose, lactose and/or dextrose.

As used herein, the term "sugar alcohol" refers to any sugar, including monosaccharides, disacharides and oligosaccharides, having a hydroxyl moiety. Sugar alcohols suitable for use in the formulations disclosed herein include mannitol, sorbitol, glycerol, xylitol, arabitol, and others, including alcohols of those sugars previously named herein. The preferred sugar alcohol is mannitol and/or sorbitol.

The term "alkalizing agent" refers to compounds, which are added to the core (i) to raise the pH 7, in order to increase the solubility of telmisartan. The alkalizing agent may be selected from one or more alkali metal hydroxides such as sodium hydroxide, potassium hydroxide, calcium hydroxide, magnesium hydroxide, ammonium hydroxide; one or more basic amino acids such as arginine, lysine; meglumine; and mixtures thereof. The preferred alkalizing agent is sodium hydroxide, meglumine and/or mixtures thereof.

The term "crystallization inhibitor" as used herein refers to an agent that inhibits, prevents, slows, delays, decreases or restricts the conversion of amorphous telmisartan or salts thereof to crystalline telmisartan or salts thereof to a measurable degree. Non-limiting examples of crystallization inhibitors include, either alone or in combination, meglumine, polyvinyl pyrrolidone, hydroxypropylmethylcellulose, etc. The preferred crystallization inhibitor is meglumine and/or polyvinyl pyrrolidone.

The term "binder" as used herein is defined as an agent able to bind particles which cannot be bound only by a compression force. The binder may be present in the pharmaceutical composition in the form of a single compound or in the form of a mixture of compounds. Non-limiting examples of binders are methylcellulose, hydroxypropyl cellulose, hydroxypropyl cellulose low substituted, hydroxyprpopyl methyl cellulose (hypromellose), macrogols, saccharose, starch, pregelatinized starch, or polyvinylpyrrolidone (povidone). The preferred binder is povidone.

The term "lubricant" as used herein is defined as an agent able to decrease adhesion of a powder to punches and friction between particles. The lubricant may be present in the pharmaceutical composition in the form of a single compound or in the form of a mixture of compounds. Non-limiting examples of lubricants are magnesium stearate, calcium stearate, zinc stearate, sodium benzoate or talc. The preferred lubricant is magnesium stearate.

Cellulose is a linear polymer of β-D-glucopyranose units joined to one another. The term "cellulose derivative" refers to cellulose that has been chemically modified from this native form. In certain embodiments of the invention the polysaccharide is a cellulose derivative such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), or hydroxypropyl methylcellulose (HPMC, hypromellose), in which one or more of the OH groups is replaced by OR, wherein R represents any of a variety of moieties. The preferred cellulose derivative is hydroxypropyl methylcellulose.

The term "diluent" as used herein is defined as an agent able to increase the bulk of a solid pharmaceutical composition, and may make a pharmaceutical dosage form containing the composition easier for the patient and/or a care giver to handle. Examples of diluents are celluloses, calcium phosphates, lactose, maltose, dextrose, polydextrose, sorbitol, mannitol, glycerol or xylitol. The preferred diluents are maltose, lactose, polydextrose and mannitol.

As used herein, the term "plasticiser" refers to a material that may be incorporated into the pharmaceutical composition in order to decrease the glass transition temperature and the melt viscosity of a polymer by increasing the free volume between polymer chains. Plasticisers include, but are not limited to citrate esters (e.g., triethylcitrate, triacetin), low molecular weight polyalkylene oxides (e.g., polyethylene glycols, polypropylene glycols, polyethylene/propylene glycols), glycerol, pentaerythritol, glycerol monoacetate, diacetate or triacetate, propylene glycol, and sodium diethyl sulfosuccinate. Additional examples of plasticisers can be found in M. & I. Ash, THE HANDBOOK OF PHARMACEUTICAL ADDITIVES (3.sup.rd ed., Synapse Information Resources, Inc., 2007).

The term "granulated" as used herein refers to an active pharmaceutical ingredient and/or an excipient present in the formulation forming a granule; defining granule as small particles gathered into a larger, permanent aggregate in which the original particles can still be identified.

The term "particle size distribution" as used herein refers to the relative percentages by volume of each of the different size fractions of a particulate matter. The particle size distributions of the present application can be measured using laser light diffraction equipment, such as Malvern Mastersizer® 2000. Particle size is determined by measuring the angular distribution of laser light scattered by a homogeneous suspension of particles. The size distribution is determined from the light scattering data using the theory of light scattering developed by Gustav Mie. Other types of equipment are also suitable to determine particle size distribution.

The term "dx", as used herein, means that x% of the particles in a composition (based on volume) have a diameter equal to or below a specified d value. Thus, a d90 of 25 µm means that 90% by volume, of the particles, have a diameter equal to or below 25 µm. In addition to using d90 as a measuring reference to determine particle size, d50 is also used for such purpose. Likewise, a d50 of 10 µm means that 50%, by volume, of the particles, have a diameter equal to or below 10 µm.

The term "micronisation" as used herein refers to the process of reducing the average diameter of a solid material's particles. Usually, the term micronisation is used when the particles that are produced are only a few micrometres in diameter. Traditional micronisation techniques are based on the use of friction to reduce particle size. Such methods include milling and grinding. Reduction in particle size may also take place as a result of collision and impact.

The term "micronised" as used herein when referring to the hydrochlorothiazide or salts thereof of the invention refers to a form of hydrochlorothiazide or salts thereof which has a particle size distribution characterised by a d₉₀ equal or below to 35 µm, preferably equal or below to 30 µm, more preferably between 30 and 10 µm.

### BRIEF DESCRIPTION OF THE INVENTION

A first aspect of the present invention relates to stable immediate release solid pharmaceutical composition of telmisartan or salts thereof and hydrochlorothiazide (HCTZ) or salts thereof, which also affords dosage uniformity. Said pharmaceutical composition comprising:
(i) a core comprising telmisartan or salts thereof;
(ii) an isolating film coating applied onto the core (i) comprising a film forming agent;
(iii) a HTZC-containing film coating applied onto the isolating film coating (ii);
   wherein the HTZC-containing film coating comprises:
   1) HTZC or salt thereof, wherein the HTZC or salt thereof have a particle size distribuition, (d₉₀), equal or below to 35 µm;
   2) a diluent;
   3) and a cellulose derivative film forming agent.

A second aspect of the present invention relates to the stable immediate release solid pharmaceutical composition of telmisartan or salts thereof and hydrochlorothiazide (HCTZ) or salts thereof, which initially contains less than 2 % (w/w) of total degradation impurities (by weight of the total weight of the active pharmaceutical ingredients) (measured by HPLC); preferably, less than 1 %. The level of the degradation impurity, 4-amino-6-chlorobenzene-1,3-disulphonamide (also known as DSA or impurity B), is also maintained below 1 % (w/w) by weight of the total weight of the active pharmaceutical ingredients (measured by HPLC); preferably, below 0.5%.

A third aspect of the present invention is to provide a process for preparing the stable immediate release solid pharmaceutical composition of telmisartan or salts thereof and hydrochlorothiazide (HCTZ) or salts thereof, which provides very good content uniformity with respect to the active ingredients. Preferably, the different samples assay carried out on the content uniformity falls between 90 to 100% of the average content.

A fourth aspect of the present invention relates to the use of the stable immediate release solid pharmaceutical composition of telmisartan or salts thereof and hydrochlorothiazide (HCTZ) or salts thereof of the first aspect of the present invention in the treatment or prophylaxis of hypertension and essential hypertension.

### DETAILED DESCRIPTION OF THE INVENTION

The present invention provides a stable immediate release solid pharmaceutical composition of telmisartan or salts thereof and hydrochlorothiazide (HCTZ) or salts thereof, which also affords dosage uniformity.

A first aspect of the present invention relates to a solid immediate release pharmaceutical composition comprising:
(i) a core comprising telmisartan or salts thereof;
(ii) an isolating film coating applied onto the core (i) comprising a film forming agent;
(iii) a HTZC-containing film coating applied onto the isolating film coating (ii);
   wherein the HTZC-containing film coating comprises:
   1) HTZC or salt thereof, wherein the HTZC or salt thereof have a particle size distribuition, (d₉₀), equal or below to 35 µm;
   2) a diluent;
   3) and a cellulose derivative film forming agent.

In a preferred embodiment the pharmaceutical composition according to the first aspect of the present invention is in the form of a tablet. In another preferred embodiment, the core of the pharmaceutical composition according to the first aspect of the present invention is a tablet core.

It has been surprisingly found that a stable and dosage-uniformed immediate release solid pharmaceutical composition of telmisartan and hydrochlorothiazide can be prepared having very low levels of total degradation products and HCTZ degradation products when the composition comprises micronised hydrochlorothiazide or salt thereof, having a particle size distribuition, (d₉₀), equal or below to 35 µm, and a cellulose derivative film forming agent in the HCTZ-containing film coating (iii). In a preferred embodiment of the first aspect of the present invention, the particle size distribuition, (d₉₀), of the hydrochlorothiazide or salt thereof is equal or below 30 µm. In a more preferred embodiment, the particle size distribuition, (d₉₀), of the hydrochlorothiazide or salt thereof is between 10 and 30 µm.

The core in accordance with the first aspect of the present invention comprises telmisartan or salt thereof from 5 to 30%, preferably from 10 to 20% by weight relative to the weight of the core (i). Additionally, the core may comprise one or more of the following pharmaceutical acceptable excipients: diluents, alkalizing agents, crystallization inhibitors, binders and lubricants (percentages expressed by weight relative to the weight of the core). The diluents may be selected from sugars and/or sugar alcohols and/or mixtures thereof, wherein the total amount of the diluent, is between 50 and 90%, preferably between 60 and 80% by weight relative to the weight of the core (i). Preferably, the diluents, contained in the core (i), can be selected from: mannitol, sorbitol, xylitol, glucose, sucrose, maltose, lactose and mixtures thereof. In a more preferred embodiment, the diluent, contained in the core is maltose, mannitol or mixtures thereof. The alkalizing agents may be selected from: sodium hydroxide, potassium hydroxide, calcium hydroxide, magnesium hydroxide, ammonium hydroxide, arginine, lysine, meglumine, and mixtures thereof. In a preferred embodiment, the alkalizing agent, contained in the core (i), is sodium hydroxide, meglumine or mixtures thereof. The alkalizing agents may be present between 2 and 10% w/w, preferably between 4% and 8% w/w by weight relative to the weight of the core (i). The crystallization inhibitors may be selected from: meglumine, polyvinyl pyrrolidone, hydroxypropylmethylcellulose and mixtures thereof. In a preferred embodiment, the crystallization inhibitor, contained in the core (i), is meglumine and/or polyvinyl pyrrolidone. The crystallization inhibitors may be present between 2 and 8% w/w, preferably between 3% and 7% w/w, by weight relative to the weight of the core (i). The binders, contained in the core (i), can be selected from: polyvinylpyrrolidone (povidone), pregelatinized starch, methylcellulose, hypromellose, hydroxypropyl cellulose, hydroxypropyl cellulose low substituted, and mixtures thereof. In a preferred embodiment, the binder, contained in the core (i), is povidone. The binders may be present between 2 and 10% w/w, preferably between 3% and 7% w/w, by weight relative to the weight of the core (i). The lubricants, contained in the core (i), may be selected from: magensium sterearate, talc, zinc stearate, calcium stearate, sodium benzoate, and mixtures thereof. In a preferred embodiment, the lubricant, contained in the core (i), is magensium sterearate. The lubricants may be present between 0.2 and 5% w/w, preferably between 0.5% and 3% w/w, by weight relative to the weight of the core (i).

The use of an isolating film coating containing a film forming agent improves the stability of the pharmaceutical composition without detriment of the suitable release of the active pharmaceutical agent contained in the core, and therefore without affecting to its bioavailability.

The isolating film coating of the pharmaceutical composition according to the first aspect of the present invention can be prepared in-house or being a commercially available pre-formulated coating material. The film forming agent used in the isolating film coating include, but is not limited to cellulose derivatives such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), or hydroxypropyl methylcellulose (HPMC, hypromellose); pre-formulated coating materials containing cellulose derivatives, such as Opadry products based on cellulose derivatives; and mixtures thereof. Preferably, the film forming agent, contained in the isolating film forming agent, is selected from HPMC and/or pre-formulated coating material containing HPMC.

The isolating film coating (ii) of the pharmaceutical composition according to the first aspect of the present invention further comprises a diluent and a plasticiser. The diluent is preferably selected from lactose, fructose, polydextrose, sacarose, maltose and mixtures thereof; more preferably the diluent is polydextrose, lactose or mixtures thereof; most preferably, the diluent is lactose. The plasticiser is preferably selected from citrate esters (e.g. triethylcitrate, triacetin); low molecular weight polyalkylene oxides (e.g. polyethylene glycols, polypropylene glycols, polyethylene/propylene glycols); glycerol, pentaerythritol, glycerol monoacetate, diacetate or triacetate, propylene glycol, and sodium diethyl sulfosuccinate. More preferably, the plasticiser is polyethylene glycol. In a preferred embodiment, the polyethylene glycol is in an amount below 15%, preferebly below 12% by weight relative to the weight of the isolating film coating. The isolating film coating can further comprises opacifiers and colorants selected from titanium dioxide, calcium carbonate, iron oxides, aluminium lakes or mixtures thereof.

The pharmaceutical composition according to the first aspect of the present invention also comprises a film coating, refers as to HCTZ-containing film coating (iii), which comprises hydrochlorothiazide or salts thereof, a diluent, and a cellulose derivative film forming agent. The diluent present in the HCTZ-containing film coating (iii) is selected from a sugar and/or sugar alcohol. Preferred diluents are selected from lactose, fructose, polydextrose, sacarose, maltose, mannitol, sorbitol, xylitol and mixtures thereof. More preferably the diluents are lactose, polydextrose or mixtures thereof. Most preferably the diluent is lactose. The percentage of the diluent in the HCTZ-containing film coating (iii) is between 8% and 40%, preferably between 10% and 32% by weight relative to the weight of the HCTZ-containing film coating (iii).

The hydrochlorothiazide or salt thereof, contained in the HCTZ-containing film coating (iii) of the first aspect of the present invention is characterised by a particle size distribuition, (d₉₀), of the hydrochlorothiazide or salt thereof equal or below to 35 µm. In a preferred embodiment, the d₉₀ of said hydrochlorothiazide or salt thereof is equal or below to 30 µm. In a more preferred embodiment, the d₉₀ of said hydrochlorothiazide or salt thereof is between 10 and 30 µm. Surprisingly, it has been found that the use of micronised hydrochlorothiazide allows preparing a stable and dosage-uniformed solid immediate release pharmaceutical composition of telmisartan and hydrochlorothiazide having very low levels of HCTZ degradation related substances. Preferably the stable and dosage-uniformed solid immediate release pharmaceutical compositions of our invention are uniform tablets, that is tablets which may be made by means of conventional tableting equipment and the uniformity is characterised in that the active substance is substantially evenly and uniformly distributed throughout the whole tablet.

The hydrochlorothiazide or salt thereof of the first aspect of the present invention can be also characterised by having a particle size distribuition, characterised by a d₅₀ equal or below to 20 µm. In a preferred embodiment, the d₅₀ of said hydrochlorothiazide or salt thereof is equal or below to 15 µm. In a more preferred embodiment, the d₅₀ of said hydrochlorothiazide or salt thereof is equal or below to 12 µm.

Moreover, the hydrochlorothiazide or salt thereof, according to the first aspect of the present invention is micronised and therefore does not have to be granulated. The percentage of hydrochlorotiazide in the HCTZ-containing film coating (iii) is between 35% and 70%, preferably between 40% and 60% by weight relative to the weight of the HCTZ-containing film coating (iii).

The film forming agents used in the HCTZ-containing film coating (iii), include but is not limited to cellulose derivatives such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), hydroxypropyl methylcellulose (HPMC, hypromellose) or pre-formulated coating materials containing cellulose derivatives, such as Opadry products based on cellulose derivatives; and mixtures thereof. Preferably, the film forming agent, contained in the HCTZ-containing film coating, is selected from HPMC and/or pre-formulated coating material containing HPMC. The percentage of cellulose derivative film forming agent in the HCTZ-containing film coating is between 15% and 35%, preferably between 18% and 30% by weight relative to the weight of the HCTZ-containing film coating (iii).

Surprisingly, it has been observed that the use of an aqueous medium for preparation of both, the isolating film coating (ii) and the HCTZ-containing film coating coating (iii), does not affect either the stability or the dosage uniformity of the pharmaceutical composition according to the present invention, with no increase of degradation related substances or change in the dosage uniformity of the hydrochlorothiazide. The present invention, nevertheless, allows the presence of organic solvents such as short-chain alcohols, for example, methanol, chlorinated derivatives such as methylene chloride, acetone or mixtures thereof with water, as a medium for preparation of both, the isolating film coating (ii) and the HCTZ-containing film coating coating (iii). The preferred said solvent medium is water.

In a particular embodiment, the pharmaceutical composition according to the first aspect of the present invention comprises from 85% to 98% (w/w) of the core (i), preferably from 88% to 96% (w/w), from 0.5% to 2.5%, (w/w) of the isolating film coating (ii), preferably from 0.7% to 1.5% (w/w), and from 1.5% to 13%, (w/w) of the HCTZ-containing film coating (iii), preferably from 3% to 10% (w/w). Weight percentage by weight relative to the weight of the pharmaceutical composition. The sum of all componets adding to a hundred.

In a more preferably embodiment of the first aspect of the present invention the solid pharmaceutical composition comprises:
(i) a core comprising telmisartan or salts thereof;
(ii) an isolating film coating applied onto the core (i) comprising a film forming agent selected from cellulose derivatives
(iii) a HCTZ-containing film coating applied onto the isolating film coating (ii),
   wherein the HTZC-containing film coating comprises:
   1) HTZC or salt thereof, wherein the HTZC or salt thereof have a particle size distribuition, (d₉₀), equal or below to 35 µm;
   2) a diluent;
   3) and a cellulose derivative film forming agent.

In a more preferably embodiment of the first aspect of the present invention the solid pharmaceutical composition comprises:
(i) a core comprising telmisartan or salts thereof;
(ii) an isolating film coating applied onto the core (i) comprising a film forming agent,
(iii) a HCTZ-containing film coating applied onto the isolating film coating (ii), wherein the HTZC-containing film coating comprises:
   1) HTZC or salt thereof, wherein the HTZC or salt thereof have a particle size distribuition, (d₉₀), equal or below to 35 µm;
   2) a diluent, selected from a sugar, a sugar alcohol or mixtures thereof;
   3) and a cellulose derivative film forming agent.

In a more preferably embodiment of the first aspect of the present invention the solid pharmaceutical composition comprises:
(i) a core comprising telmisartan or salts thereof and maltose as a diluent
(ii) an isolating film coating applied onto the core (i) comprising a film forming agent;
(iii) a HCTZ-containing film coating applied onto the isolating film coating (ii), wherein the HTZC-containing film coating comprises:
   1) HTZC or salt thereof, wherein the HTZC or salt thereof have a particle size distribuition, (d₉₀), equal or below to 35 µm;
   2) a diluent;
   3) and a cellulose derivative film forming agent;

In a more preferably embodiment of the first aspect of the present invention the solid pharmaceutical composition comprises:
(i) a core comprising telmisartan or salts thereof;
(ii) an isolating film coating applied onto the core (i) comprising a film forming agent;
(iii) a HCTZ-containing film coating applied onto the isolating film coating (ii), wherein the HTZC-containing film coating comprises:
   1) HTZC or salt thereof, wherein the HTZC or salt thereof have a particle size distribuition, (d₉₀), equal or below to 35 µm;
   2) a diluent;
   3) and a cellulose derivative film forming agent
(iv) optionally, further comprising an external film coating that is applied onto the HCTZ-containing film coating (iii).

Said external film coating may be used in order to further prevents loss of any ingredient of the already functionally complete dosage form and to improve the appearance of the solid pharmaceutical composition. The external film coating of the pharmaceutical composition according to this embodiment can be prepared in-house or being a commercially available pre-formulated coating material. Said external film coating comprises a film forming agent, which can be any polymer appropriate for film coating. Examples of appropriate polymers/film forming agents include, but are not limited to cellulose derivatives such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), or hydroxypropyl methylcellulose (HPMC, hypromellose); acrylics such as methacrylate; or methyl methacrylate copolymers; and vinyls such a as polyvinyl alcohol. Preferably, the film forming agent, contained in the external film coating, is a cellulose derivative or a vinyl. The external film coating of the pharmaceutical composition according to the first aspect of the present invention further comprises a diluent and a plasticiser. The diluent is preferably selected from lactose, fructose, polydextrose, sacarose, maltose and mixtures thereof; more preferably the diluent is polydextrose, lactose or mixtures thereof; most preferably, the diluent is lactose. The plasticiser is preferably selected from citrate esters (e.g. triethylcitrate, triacetin); low molecular weight polyalkylene oxides (e.g. polyethylene glycols, polypropylene glycols, polyethylene/propylene glycols); glycerol, pentaerythritol, glycerol monoacetate, diacetate or triacetate, propylene glycol, and sodium diethyl sulfosuccinate. More preferably, the plasticiser is polyethylene glycol. The external film coating may further comprise opacifiers and colorants selected from titanium dioxide, calcium carbonate, iron oxides, aluminium lakes or mixtures thereof.

A second aspect of the present invention relates to the stable immediate release solid pharmaceutical composition of telmisartan or salts thereof and hydrochlorothiazide (HCTZ) or salts thereof, as described in the first aspect of the present invention, which initially contains less than 2 % (w/w) of total degradation impurities (by weight of the total weight of the active pharmaceutical ingredients) (measured by HPLC); preferably, less than 1 %. The level of the degradation impurity, 4-amino-6-chlorobenzene-1,3-disulphonamide (also known as DSA or impurity B), is also maintained below 1 % (w/w) by weight of the total weight of the active pharmaceutical ingredients (measured by HPLC); preferably, below 0.5%.

The stability of the pharmaceutical composition of telmisartan or salts thereof and hydrochlorothiazide (HCTZ) or salts thereof, as described in the first aspect of the present invention, was evaluated and calculated according to the pharmaceutical guideline ICH Q1A (R2) "Stability Testing of New Drug Substances and Products" of February 2003. The effect of temperature and storage conditions may be tested. The stability tests are preferably performed initially (right after manufacturing) and after 1 month of storage of the pharmaceutical composition of the present invention. The pharmaceutical compositions of the present invention were packed in aluminium foil blisters (on both sides). Tested storage conditions were 40°C ± 2°C temperature and 75 % ± 5% RH.

The present inventors have found that the pharmaceutical compositions of the invention, initially containing less than 2% (w/w), preferably less than 1% (w/w), of total degradation impurities (measured by HPLC) (by weight of the total weight of the active pharmaceutical ingredients), were stable and kept the level of total degradation impurities (including 4-amino-6-chlorobenzene-1,3-disulphonamide) below 1% (w/w), preferably below 0.5% (w/w) by weight of the total weight of the active pharmaceutical ingredients after 1 month at 40°C/75% HR. The level of the degradation impurity, 4-amino-6-chlorobenzene-1,3-disulphonamide, is also maintained below 0.3% (w/w), preferably below 0.2% (w/w) by weight of the total weight of the active pharmaceutical ingredients after 1 month at 40°C/75% HR.

The inventors of the present invention have surprisingly found that the use of the isolating film coating (ii) comprising a film forming agent; preferably cellulose derivatives, between the core (i), which include alkaline agents; and the HCTZ-containing film coating (iii) provides stable pharmaceutical compositions that avoids the degradation of hydrochlorothiazide, which was confirmed by the stability tests performed.

A third aspect of the present invention is to provide a process for preparing the stable immediate release solid pharmaceutical composition of telmisartan or salts thereof and hydrochlorothiazide (HCTZ) or salts thereof of the first aspect of the present invention, which provides very good content uniformity with respect to the active ingredients. Preferably, the different samples assay carried out on the content uniformity falls between 90 to 100% of the average content. The process for the preparation of stable and dosage-uniformed solid pharmaceutical compositions of telmisartan or salts thereof and hydrochlorothiazide (HCTZ) or salts thereof, described in the first embodiment, comprises the following steps:
(1) providing a core (i), which comprises the steps of:
   a) preparing a granulation fluid by dissolving an alkalizing agent in water, followed by addition of telmisartan and a binder,
   b) spraying the granulation fluid obtained in step a) onto a diluent, to obtain a granulate; wherein the diluent is optionally granulated
   c) drying and sieving the granules obtained in step b),
   d) mixing the dried granules obtained in step c) with a diluent, wherein diluent is optionally granulated
   e) mixing a lubricant with the blend obtained in step d),
   f) compressing the blend obtained in step e) to form a core,
(2) coating the core obtained in step (1) with an isolating film coating comprising a film forming agent,
(3) coating the coated core obtained in step (2); with a film coating comprising a mixture of hydrochlorothiazide or salts thereof, a diluent and a film forming agent.
(4) optionally, further coating the coated core obtained in step (3) with an external film coating.

The hydrochlorothiazide or salt thereof used in step (3), according to the third aspect of the present invention, is micronised and therefore does not have to be granulated.

The coating process of steps (2), (3) and/or (4), according to the third aspect of the present invention, may be performed by using conventional coating procedures and equipment, such as spray or spray-drying by means of coating pan, spray or fluidized coating equipment. Preferably said coating process is performed by spray-drying using a coating pan.

A fourth aspect of the present invention relates to the use of the stable immediate release solid pharmaceutical composition of telmisartan or salts thereof and hydrochlorothiazide (HCTZ) or salts thereof of the first aspect of the present invention in the treatment or prophylaxis of hypertension and essential hypertension.

It will be readily apparent to one skilled in the art that varying substitutions and compositions may be made to the invention disclosed herein without departing from the scope and spirit of the invention. Thus, it should be understood that although the present invention has been specifically disclosed by preferred embodiments and optional features, modifications and variations of the concepts herein disclosed may be resorted to by those skilled in the art, and that such modifications and variations are considered to be falling within the scope of the invention.

Further aspects/embodiments of the present invention can be found in the following clauses:
1. A solid immediate release pharmaceutical composition comprising:
   (i) a core comprising telmisartan or salts thereof;
   (ii) an isolating film coating applied onto the core (i) comprising a film forming agent;
   (iii) a HTZC-containing film coating applied onto the isolating film coating (ii);
      wherein the HTZC-containing film coating comprises:
      1) HTZC or salt thereof, wherein the HTZC or salt thereof have a particle size distribuition, (d₉₀), equal or below to 35 µm;
      2) a diluent;
      3) and a cellulose derivative film forming agent.
2. The solid pharmaceutical composition according to clause 1, wherein the particle size distribuition, (d₉₀), of the hydrochlorothiazide or salt thereof is equal or below to 30 µm.
3. The solid pharmaceutical composition according to clause 1, wherein the particle size distribuition, (d₉₀), of the hydrochlorothiazide or salt thereof is between 10 and 30 µm.
4. The solid pharmaceutical composition according to any of the preceding clauses, wherein the pharmaceutical composition is in the form of a tablet.
5. The solid pharmaceutical composition according to any one of the preceeding clauses wherein the core is a tablet core.
6. The solid pharmaceutical composition according to any of the preceding clauses, wherein the core (i) comprises from 5 to 30%, preferably from 10 to 20% of telmisartan or salt thereof by weight relative to the weight of the core (i).
7. The solid pharmaceutical composition according to the preceding clause 6, wherein the diluent of the core (i) is selected from a sugar, a sugar alcohol and mixtures thereof.
8. The solid pharmaceutical composition according to the preceding clause 6, wherein the diluent of the core (i) is preferably selected from mannitol, sorbitol, xylitol, glucose, dextrose, sucrose, maltose, lactose and mixtures thereof.
9. The solid pharmaceutical composition according to the preceding clause, wherein the preferred diluent of the core (i) is maltose, mannitol or mixtures thereof.
10. The solid pharmaceutical composition according to any one of the preceding clauses, wherein the total amount of the diluent of the core (i), is between 50 and 90%, preferably between 60 and 80% by weight relative to the weight of the core (i).
11. The solid pharmaceutical composition according to any of the preceding clauses, wherein the core (i) comprises an alkalizing agent preferably selected from sodium hydroxide, potassium hydroxide, calcium hydroxide, magnesium hydroxide, ammonium hydroxide, arginine, lysine, meglumine, and mixtures thereof.
12. The solid pharmaceutical composition according to the preceding clause, wherein the preferred alkalizing agent of the core (i) is sodium hydroxide, meglumine or mixtures thereof.
13. The solid pharmaceutical composition according to any of the preceding clauses, wherein the percentage of alkalizing agent is between 2 and 10%, preferably between 4 and 8% by weight relative to the weight of the core (i).
14. The solid pharmaceutical composition according to any of the preceding clauses, wherein the core (i) comprises a crystallization inhibitor selected from meglumine, polyvinyl pyrrolidone, hydroxypropylmethylcellulose and mixtures thereof.
15. The solid pharmaceutical composition according to the preceding clause, wherein the preferred crystallization inhibitor of the core (i) is meglumine and/or polyvinyl pyrrolidone.
16. The solid pharmaceutical composition according to any of the preceding clauses, wherein the percentage of crystallization inhibitor is between 2 and 8%, preferably between 3 and 7% by weight relative to the weight of the core (i).
17. The solid pharmaceutical composition according to any of the preceding clauses, wherein the core (i) comprises a binder, preferably selected from polyvinylpyrrolidone (povidone), pregelatinized starch, methylcellulose, hypromellose, hydroxypropyl cellulose, hydroxypropyl cellulose low substituted, and mixtures thereof.
18. The solid pharmaceutical composition according to the preceding clause, wherein the preferred binder of the core (i) is povidone.
19. The solid pharmaceutical composition according to any of the preceding clauses, wherein the percentage of binder is between 2 and 10%, preferably between 3 and 7% by weight relative to the weight of the core (i).
20. The solid pharmaceutical composition according to any of the preceding clauses, wherein the core (i) comprises a lubricant, preferably selected from magensium sterearate, talc, zinc stearate, calcium stearate, sodium benzoate, and mixtures thereof.
21. The solid pharmaceutical composition according to the preceding clause, wherein the preferred lubricant of the core (i) is magnesium steareate.
22. The solid pharmaceutical composition according to any of the preceding clauses, wherein the percentage of lubricant is between 0.2 and 5%, preferably between 0.5 and 3% by weight relative to the weight of the core (i).
23. The solid pharmaceutical composition according to any of the preceding clauses, wherein the isolating film coating (ii) comprises a film forming agent preferably selected from methyl cellulose, hydroxymethyl cellulose, hydroxyethyl cellulose, hydroxypropyl cellulose, hydroxypropyl methylcellulose, sodium carboxymethyl cellulose, and mixtures thereof.
24. The solid pharmaceutical composition according to the preceding clause, wherein the film forming agent contained in the isolating film coating (ii) is more preferably hydroxypropyl methylcellulose.
25. The solid pharmaceutical composition according to any of the preceding clauses, wherein the isolating film coating (ii) further comprises a diluent, selected from lactose, fructose, polydextrose, sacarose, maltose and mixtures thereof.
26. The solid pharmaceutical composition according to the preceding clause, wherein the diluent of the isolating film coating (ii) is preferably lactose or polydextrose, more preferably lactose.
27. The solid pharmaceutical composition according to any of the preceding clauses, wherein the isolating film coating (ii) further comprises a plasticiser selected from citrate esters, selected from triethylcitrate, triacetin; low molecular weight polyalkylene oxides selected from polyethylene glycols, polypropylene glycols, polyethylene/propylene glycols; glycerol, pentaerythritol, glycerol monoacetate, diacetate or triacetate, propylene glycol, and sodium diethyl sulfosuccinate.
28. The solid pharmaceutical composition according to the preceding clause, wherein the plasticiser of the isolating film coating (ii) is preferably polyethylene glycol.
29. The solid pharmaceutical composition according to the two preceding clauses, wherein the percentage of polyethylene glycol of the isolating film coating (ii) is below 15%, preferebly below 12% by weight relative to the weight of the isolating film coating (ii).
30. The solid pharmaceutical composition according to any of the preceding clauses, wherein the HCTZ-containing film coating (iii) comprises a cellulose derivative selected from methyl cellulose, hydroxymethyl cellulose, hydroxyethyl cellulose, hydroxypropyl cellulose, hydroxypropyl methylcellulose, sodium carboxymethyl cellulose and mixtures thereof.
31. The solid pharmaceutical composition according to the preceding clause, wherein the cellulose derivative film forming agent contained in the HCTZ-containing film coating (iii) is preferably hydroxypropyl methylcellulose.
32. The solid pharmaceutical composition according to any of the preceding clauses, wherein the diluent of the HCTZ-containing film coating (iii) is selected from a sugar, a sugar alcohol or mixtures thereof.
33. The solid pharmaceutical composition according to any of the preceding clauses, wherein the diluent of the HCTZ-containing film coating (iii) is preferably selected from lactose, fructose, polydextrose, sacarose, maltose, mannitol, sorbitol, xylitol and mixtures thereof.
34. The solid pharmaceutical composition according to the preceding clause, wherein the diluent of the HCTZ-containing film coating (iii) is preferably lactose or polydextrose, more preferably lactose.
35. The solid pharmaceutical composition according to any of the preceding clauses, wherein the percentage of hydrochlorotiazide in the HCTZ-containing film coating (iii) is between 35% and 70%, preferably between 40% and 60% by weight relative to the weight of the hydrochlorothiazide film coating (iii).
36. The solid pharmaceutical composition according to any of the preceding clauses, wherein the hydrochlorotiazide does not have to be granulated.
37. The solid pharmaceutical composition according to any of the preceding clauses, wherein the hydrochlorotiazide is micronised.
38. The solid pharmaceutical composition according to any preceding clauses, wherein the the percentage of diluent in the HCTZ-containing film coating (iii) is between 8% and 40%, preferably between 10% and 32% by weight relative to the weight of the HCTZ-containing film coating (iii).
39. The solid pharmaceutical composition according to any of the preceding clauses, wherein the the percentage of cellulose derivative film forming agent in the HCTZ-containing film coating (iii) is between 15% and 35%, preferably between 18% and 30% by weight relative to the weight of the HCTZ-containing film coating (iii).
40. The solid pharmaceutical composition according to any of the preceding clauses, wherein the core (i) constitutes from 85% to 98%, preferably from 88% to 96% by weight relative to the weight of the solid pharmaceutical composition.
41. The solid pharmaceutical composition according to any of the preceding clauses, wherein the isolating film coating (ii) constitutes 0.5% to 2.5%, preferably from 0.7% to 1.5% by weight relative to the weight of the solid pharmaceutical composition.
42. The solid pharmaceutical composition according to any of the preceding clauses, wherein the HCTZ-containing film coating (iii) constitutes from 1.5% to 13%, preferably from 3% to 10% by weight relative to the weight of the solid pharmaceutical composition.
43. The solid pharmaceutical composition according to any of the preceding clauses, wherein an external film coating is applied onto a HCTZ-containing film coating (iii)
44. The solid pharmaceutical composition according to any of the preceding clauses which initially contains equal or less than 2% (w/w) of the total degradation impurities, as measured by HPLC.
45. The solid pharmaceutical composition according to any of the preceding clauses, which initially contains equal or less than 1% (w/w) of the total degradation impurities as measured by HPLC.
46. The solid pharmaceutical composition according to any of the preceding clauses, wherein the level of total degradation impurities, including 4-amino-6-chlorobenzene-1,3-disulphonamide, is maintained equal or below 2% (w/w) after 1 month at 40°C/75% HR, as measured by HPLC.
47. The solid pharmaceutical composition according to any of the preceding clauses, wherein the level of total degradation impurities, including 4-amino-6-chlorobenzene-1,3-disulphonamide, is maintained equal or below 1% (w/w) after 1 month at 40°C/75% HR, as measured by HPLC.
48. The solid pharmaceutical composition according to any of the preceding clauses, wherein the level of impurity 4-amino-6-chlorobenzene-1,3-disulphonamide, is maintained equal or below 1% (w/w) after 1 month at 40°C/75% HR, as measured by HPLC.
49. The solid pharmaceutical composition according to any of the preceding clauses, wherein the level of impurity 4-amino-6-chlorobenzene-1,3-disulphonamide, is maintained equal or below 0.5% (w/w) after 1 month at 40°C/75% HR, as measured by HPLC.
50. A process for preparing a solid immediate release pharmaceutical composition comprising the steps of:
   (1) providing a core (i), which comprises the steps of:
      a) preparing a granulation fluid by dissolving an alkalizing agent in water, followed by addition of telmisartan and a binder,
      b) spraying the granulation fluid obtained in step a) onto a diluent, to obtain a granulate; wherein the diluent is optionally granulated
      c) drying and sieving the granules obtained in step b),
      d) mixing the dried granules obtained in step c) with a diluent; wherein the diluent is optionally granulated
      e) mixing a lubricant with the blend obtained in step d),
      f) compressing the blend obtained in step e) to form a core,
   (2) coating the core obtained in step (1) with an isolating film coating comprising a film forming agent,
   (3) coating the coated core obtained in step (2); with a film coating comprising a mixture of hydrochlorothiazide or salts thereof, a diluent and a film forming agent.
   (4) optionally, further coating the coated core obtained in step (3) with an external film coating.
51. A process acoording to the preceeding clause wherein, the diluent of step (1) is selected from sugars, sugar alcohols or mixtures thereof.
52. A process according to the two preceeding clauses, wherein the diluent of step (1) is preferably selected form mannitol, sorbitol, xylitol, glucose, sucrose, maltose, lactose and mixtures thereof.
53. A process according to the three preceeding clauses, wherein the diluent of step (1) is more preferably maltose, mannitol or mixtures thereof.
54. A process according to clauses 50 to 53, wherein the binder of step (1) is selected from polyvinylpyrrolidone (povidone), pregelatinized starch, methylcellulose, hypromellose, hydroxypropyl cellulose, hydroxypropyl cellulose low substituted, and mixtures thereof.
55. A process according to the preceeding clause, wherein the binder of step (1) is preferably povidone.
56. A process according to clauses 50 to 55, wherein the film forming agent of step (2) is selected from cellulose derivate.
57. A process according to the preceeding clause, wherein the film forming agent of step (2) is preferably selected from methyl cellulose, hydroxymethyl cellulose, hydroxyethyl cellulose, hydroxypropyl cellulose, hydroxypropyl methylcellulose, sodium carboxymethyl cellulose, and mixtures thereof
58. A process according to the two preceeding clauses, wherein the film forming agent of step (2) is more preferably hydroxypropyl methylcellulose.
59. A process according to clauses 50 to 58, wherein the diluent of step (3) is preferably selected from lactose, fructose, polydextrose, sacarose, maltose, mannitol, sorbitol, xylitol and mixtures thereof.
60. A process according to the preceding clause, wherein the diluent of step (3) is preferably lactose, polydextrose or mixtures thereof. Most preferably, the diluent is lactose.
61. A process according to clauses 50 to 60, wherein the film forming agent of step (3) is selected from cellulose derivate.
62. A process according to the preceeding clause, wherein the film forming agent of step (3) is preferably selected form methyl cellulose, hydroxymethyl cellulose, hydroxyethyl cellulose, hydroxypropyl cellulose, hydroxypropyl methylcellulose, sodium carboxymethyl cellulose and mixtures thereof.
63. A process according to the two preceeding clauses wherein, film forming agent of step (3) is preferably hydroxypropyl methylcellulose
64. A process according to clauses 50 to 63, wherein the coating of steps (2), (3) and (4) is performed by spraying or spray-drying. Preferably the coating is performed by spray-drying.
65. A process according to clauses 50 to 63, wherein the coating of steps (2), (3) and (4) is performed by using a coating pan, or spray or fluidized coating equipment. Preferably the coating is performed by using a coating pan.
66. A process according to clauses 50 to 65, wherein the hydrochlorotiazide or salts thereof of step (3) is micronised.
67. A process according to clauses 50 to 66, wherein the hydrochlorotiazide or salts thereof of step (3) is characterized by having a particle size distribuition, characterised by a d₉₀ equal or below to 35 µm.
68. A process according to clauses 50 to 66, wherein the particle size distribution of the hydrochlorotiazide or salts thereof of step (3) is preferably characterized by a d₉₀ equal or below to 30 µm.
69. A process according to clauses 50 to 66, wherein the particle size distribution of the hydrochlorotiazide or salts thereof of step (3) is most preferably preferably characterized by a d₉₀ between 30 and 10 µm.
70. The solid pharmaceutical composition according to clauses 1 to 49, for the treatment of hypertension and essential hypertension.

In the following, the present invention is further illustrated by examples. They should in no case be interpreted as a limitation of the scope of the invention as defined in the claims. Unless indicated otherwise, all indications of percentage are by weight and temperatures are in degrees Celsius.

### EXAMPLES

### Example 1

### Formulation composition

| **Ingredients** | **Quantities** |
|---|---|
| **Tablet core** | **mg/Tablet** |
| Telmisartan | 80,0 |
| Sodium hydroxide | 6,7 |
| Meglumine | 24,0 |
| Povidone K-30 | 24,0 |
| Mannitol (granulated) | 337,3 |
| Maltose (CD grade) | 50,0 |
| Magnesium stearate | 8,0 |
| Purified water* | 270,0 |
| Total tablet core | 530,0 |

| **Isolating film coating** | |
|---|---|
| Opadry OY-L-28900 White** | 5,0 |
| Purified water* | 45,0 |

| **HCTZ-containing film coating** | |
|---|---|
| Hydrochlorotiazide (d₉₀=25 µm) | 25,0 |
| Lactose | 8,3 |
| Methocel E5 (HPMC) | 8,3 |
| Purified water* | 305,6 |
| **Total tablet** | **576,6** |

| | |
|---|---|
| *Evaporates during manufacturing process* *** Opadry OY-L-28900 White is a dry dispersion for film coating, based on hydroxypropyl methylcellulose (HPMC)* | |

### Manufacturing process

### A) Production of telmisartan core

1. A granulation fluid was prepared by dissolving sodium hydroxide and meglumine in purified water, followed by addition of telmisartan and povidone.
2. 309.28 mg of granulated mannitol were sieved (mesh size 0.5-0.6 mm) and placed in a fluid bed granulator.
3. The granulation fluid of step 1 was sprayed onto the granulated mannitol obtained in step 2.
4. The granules obtained in step 3 were dried and sieved to provide a mesh size of 1.2-1.5 mm.
5. 28,0 mg of granulated mannitol and maltose were sieved to provide a mesh size of 0.5-0.6 mm and added to the granules obtained in step 4.
6. Magnesium stearate was passed through a sieve to provide a mesh size of 0.32 mm and blended with the blend obtained in step 5.
7. The blend obtained in step 6 was compressed to provide a tablet core.

### B) Isolating film coating

8. An aqueous dispersion of Opadry OY-L-28900 White was prepared and used to coat the telmisartan core provided in step A. Said coating process was performed by spray-drying using a coating pan.

### C) HCTZ-containing film coating

9. A mixture of hydrochlorothiazide (d₉₀= 25 µm), Methocel E5 (hydroxypropyl methylcellulose) and lactose was prepared.
10. An aqueous dispersion of the mixture obtained in step 9 was prepared and used to further coat the coated telmisartan core generated in step B. Said coating process was performed by spray-drying using a coating pan.

### Stability assays

The coated tablets, obtained by means of the manufacturing process described above, were packed in blister packaging made of aluminum foil on both sides and exposed to stress stability testing at 40 °C and 75% relative humid (RH) for one month. Results of the stability studies are shown below:

| **Impurities** (% relative to HCTZ content) | Initially | 1 month 40°C/75% HR |
|---|---|---|
| DSA (%) | >0,1 | 0,2 |
| Total degradation products % | >0,1 | 0,2 |

## Claims

1. A solid immediate release pharmaceutical composition comprising:
(i) a core comprising telmisartan or salts thereof;
(ii) an isolating film coating applied onto the core (i) comprising a film forming agent;
(iii) a HTZC-containing film coating applied onto the isolating film coating (ii);
wherein the HTZC-containing film coating comprises:
1) HTZC or salt thereof, wherein the HTZC or salt thereof have a particle size distribuition, (d₉₀), equal or below to 35 µm;
2) a diluent;
3) and a cellulose derivative film forming agent.

2. The solid pharmaceutical composition according to claim 1, wherein the particle size distribuition, (d₉₀), of the hydrochlorothiazide or salt thereof is equal or below to 30 µm.

3. The solid pharmaceutical composition according to any of the preceeding claims wherein the core is a tablet core

4. The solid pharmaceutical composition according to any of the preceding claims, wherein the core (i) comprises from 5 to 30%, preferably from 10 to 20% of telmisartan or salt thereof by weight relative to the weight of the core (i).

5. The solid pharmaceutical composition according to any of the preceding claims, wherein the core (i) comprises a diluent, selected from a sugar, a sugar alcohol and mixtures thereof.

6. The solid pharmaceutical composition according to any of the preceding claims, wherein the core (i) comprises a diluent selected from mannitol, sorbitol, xylitol, glucose, sucrose, maltose, lactose and mixtures thereof; preferebly the diluent is selected from maltose, mannitol and mixtures thereof.

7. The solid pharmaceutical composition according to any of the preceding claims, wherein the core (i) comprises a binder, preferably selected from polyvinylpyrrolidone (povidone), pregelatinized starch, methylcellulose, hypromellose, hydroxypropyl cellulose, hydroxypropyl cellulose low substituted, and mixtures thereof.

8. The solid pharmaceutical composition according to any of the preceding claims, wherein the isolating film coating (ii) comprises a film forming agent preferably selected from methyl cellulose, hydroxymethyl cellulose, hydroxyethyl cellulose, hydroxypropyl cellulose, hydroxypropyl methylcellulose, sodium carboxymethyl cellulose and mixtures thereof.

9. The solid pharmaceutical composition according to the preceding claim, wherein the film forming agent contained in the isolating film coating (ii) is hydroxypropyl methylcellulose.

10. The solid pharmaceutical composition according to any of the preceding claims, wherein the isolating film coating (ii) further comprises a diluent, preferably selected from lactose, fructose, polydextrose, sacarose and mixtures thereof.

11. The solid pharmaceutical composition according to any of the preceding claims, wherein the isolating film coating (ii) further comprises a plasticiser selected from citrate esters, selected from triethylcitrate, triacetin; low molecular weight polyalkylene oxides selected from polyethylene glycols, polypropylene glycols, polyethylene/propylene glycols; glycerol, pentaerythritol, glycerol monoacetate, diacetate or triacetate, propylene glycol, and sodium diethyl sulfosuccinate; more preferably the plasticiser is polyethelene glycol.

12. The solid pharmaceutical composition according to any of the preceding claims, wherein the HCTZ-containing film coating (iii) comprises a cellulose derivative preferably selected from methyl cellulose, hydroxymethyl cellulose, hydroxyethyl cellulose, hydroxypropyl cellulose, hydroxypropyl methylcellulose, sodium carboxymethyl cellulose and mixtures thereof; more preferably the film forming agent is hydroxypropyl methylcellulose.

13. The solid pharmaceutical composition according to any of the preceding claims, wherein the percentage of hydrochlorotiazide in the HCTZ-containing film coating (iii) is between 35% and 70%, preferably between 40% and 60% weight relative to the weight of the hydrochlorothiazide film coating (iii).

14. The solid pharmaceutical composition according to any of the preceding claims, wherein the the percentage of cellulose derivative film forming agent in the hydrochlorothiazide film coating (iii) is between 15% and 35%, preferably between 18% and 30% by weight relative to the weight of the HCTZ-containing film coating (iii).

15. The solid oral pharmaceutical composition according to any of the preceding claims, wherein the level of total degradation impurities, including 4-amino-6-chlorobenzene-1,3-disulphonamide, is maintained equal or below 2% (w/w) after 1 month at 40°C/75% HR, as measured by HPLC.

16. The solid oral pharmaceutical composition according to any of the preceding claims, wherein the level of total degradation impurities, including 4-amino-6-chlorobenzene-1,3-disulphonamide, is maintained equal or below 1% (w/w) after 1 month at 40°C/75% HR, as measured by HPLC.

17. A process for preparing a solid pharmaceutical composition comprising the steps of:
(1) providing a core (i), which comprises the steps of:
a) preparing a granulation fluid by dissolving an alkalizing agent in water, followed by addition of telmisartan and a binder,
b) spraying the granulation fluid obtained in step a) onto a diluent, to obtain a granulate; wherein the diluent is optionally granulated
c) drying and sieving the granules obtained in step b),
d) mixing the dried granules obtained in step c) with a diluent; wherein the diluent is optionally granulated
e) mixing a lubricant with the blend obtained in step d),
f) compressing the blend obtained in step e) to form a core,
(2) coating the core obtained in step (1) with an isolating film coating comprising a film forming agent,
(3) coating the coated core obtained in step (2); with a film coating comprising a mixture of hydrochlorothiazide or salts thereof, a diluent and a film forming agent.
(4) optionally, further coating the coated core obtained in step (3) an external film coating.

18. A process according to claim 17, wherein the film forming agent of step (2) is preferably selected from methyl cellulose, hydroxymethyl cellulose, hydroxyethyl cellulose, hydroxypropyl cellulose, hydroxypropyl methylcellulose, sodium carboxymethyl cellulose and mixtures thereof, more preferably the film forming agent is hydroxypropyl methylcellulose.

19. A process according to claims 17 and 18, wherein the coating of step (2), (3) and (4) is performed by spray-drying.

20. A process according to claims 17 to 19, wherein the hydrochlorotiazide or salts thereof of step (3) is micronised.

21. A process according to claims 17 to 20, wherein the hydrochlorotiazide or salts thereof of step (3) is **characterized by** having a particle size distribuition, **characterised by** a d₉₀ equal or below to 35 µm.

22. The solid pharmaceutical composition according to claims 1 to 16, for the treatment of hypertension and essential hypertension.
